(19) Europäisches Patentamt
European Patent Office
Office européen des brevets

(11) Publication number: **0 116 903**
**B1**

(12) **EUROPEAN PATENT SPECIFICATION**

(45) Date of publication of patent specification: **14.09.88**

(21) Application number: **84101316.2**

(22) Date of filing: **09.02.84**

(51) Int. Cl.⁴: **C 07 C 31/135**, C 07 C 69/24, C 07 C 67/08, C 07 C 67/03, A 23 L 1/226, A 24 B 3/12 // C07C29/14, C07C29/17

(54) **Flavour compositions containing campholenic alcohol or derivatives thereof.**

(30) Priority: **16.02.83 US 466820**

(43) Date of publication of application:
**29.08.84 Bulletin 84/35**

(45) Publication of the grant of the patent:
**14.09.88 Bulletin 88/37**

(84) Designated Contracting States:
**CH DE FR GB LI NL**

(56) References cited:
**FR-A-2 330 666**
**FR-A-2 335 244**
**GB-A-2 024 208**
**US-A-3 632 633**
**US-A-3 978 009**

(73) Proprietor: **L. GIVAUDAN & CIE Société Anonyme**
**CH-1214 Vernier-Genève (CH)**

(72) Inventor: **Naipawer, Richard E.**
**9 Iris Lane**
**Wallington, N.J. (US)**
Inventor: **Rohr, Martin**
**14 Cedar Street**
**Glen Rock, N.J. (US)**
Inventor: **Potter, Richard H.**
**262 Springfield Avenue**
**Hasbrouck Heights, N.J. (US)**

(74) Representative: **Urech, Peter, Dr. et al**
**Grenzacherstrasse 124 Postfach 3255**
**CH-4002 Basel (CH)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 116 903 B1

Courier Press, Leamington Spa, England.

**Description**

The present invention discloses compounds of the general formula

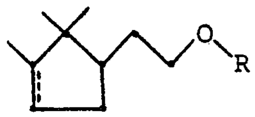

I

wherein R is hydrogen or an alkanoyl group of two to five carbon atoms and the dotted line is an optional bond, which compounds are valuable flavour compounds and can be used to improve the flavour of foodstuffs and luxury consumables. The invention also discloses methods for preparing such compositions.

The art of creating flavours involves blending a number of substances having individual characteristics to produce a composition which has the desired flavour. A successful product is not simply a combination of pleasant tasting materials; a successful product is one in which the individual character of each of the components is not readily perceived per se, but blends with each of the other flavour notes to provide a single organoleptic impression.

To create this single organoleptic impression, the flavourist uses a number of compounds which not only contribute their own characteristic flavour to the blend, but which tie together the other materials used in the composition to form a more uniformly blended composition. To meet the demands of the flavourist, such a chemical must not only tie together the individual contributions of the other materials, but must do it in such a way that the resulting flavour more closely resembles the natural flavour. This latter ability is described by the flavourist as the ability to add "naturalness" to the composition. There is always a need for compounds which have this ability.

The present invention concerns flavour compositions, foodstuffs and luxury consumables (tobacco, chewing gum, etc.) wherein the flavour has been improved, enhanced or modified by the addition of a compound of the formula

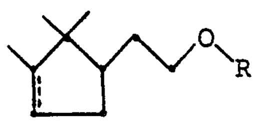

I

wherein R is hydrogen or an alkanoyl group of from two to five carbon atoms and the dotted line represents an optional bond.

The compounds of formula I are characterized by organoleptic properties that make them especially useful in flavour compositions. Although several of the compounds represented by formula I are known, there is no mention of their organoleptic properties in the prior art.

The compound of formula I wherein R is hydrogen and the dotted line represents an additional bond is known as 2-(2,2,3-trimethylcyclopent-3-en-1-yl)ethanol, or more commonly as α-campholenic alcohol. The compounds used in this invention will be referred to as derivatives of α-campholenic alcohol.

The compounds of formula I wherein R is hydrogen or acetyl are known; those wherein R is an alkanoyl group of from three to five carbon atoms are novel, and, as well as their preparation, form also part of the present invention.

Furthermore, polysubstituted cyclopentene derivatives bearing functional groups in the 1-side chain further remote from the ring are described in GB—A—2 024 208. The compounds are sandalwood odorants.

The alcohols and esters used in this invention may be conveniently prepared by synthetic methods which are generally known in the art. The α-campholenic alcohol for example, may be prepared by a metal hydride reduction of α-campholenic aldehyde. Both the (R)- and (S)-alcohols were made by reducing the corresponding (R)- and (S)-α-campholenic aldehydes prepared in accord with the procedures described in U.S. Patent 4,052,341. No percepticle differences were noted in the flavours of the two antipodes and the (R)-isomer, the (S)-isomer or enantiomeric mixtures can be used interchangeably.

Dihydro-α-campholenic alcohol can be prepared from the α-campholenic alcohol by hydrogenation of the double bond, from the dihydro-α-campholenic aldehyde by reduction of the carbonyl group, or from α-campholenic aldehyde by reduction of the carbon-carbon double bond and carbonyl group simultaneously. All three approaches are within the ordinary skill of a chemist. The process employed herein is the hydrogenation of α-campholenic alcohol using a metal catalyst (e.g. 5% palladium on carbon.).

The esters of α-campholenic alcohol and dihydro-α-campholenic alcohol may be prepared by general esterification methods known in the art for producing esters of primary alcohols. Thus, the esters are obtainable by reacting such primary alcohols with carboxylic acids or derivatives of these carboxylic acids, e.g. carboxylic acid halides or anhydrides, etc; furthermore, the esters are also obtainable by transesterification methods. Suitable methods are provided in the examples.

Transesterification methods would involve reacting any acetyl, propanoyl, butanoyl, pentanoyl, etc. ester, for example the methyl ester, with either α-campholenic alcohol or the dihydro-α-campholenic

alcohol. This is essentially an alcohol exchange and the reaction parallels that of reacting an acid and an alcohol except that in this case the by-product is an alcohol rather than water.

While such reactions are usually carried out using catalytic amounts of an acid or a base catalyst, some reaction can take place, albeit more slowly, without a catalyst.

It is usually preferred to use a small amount of an alkoxide as the catalyst. The amount is not critical and can typically vary from 0.1 to 0.001 moles/mole of the alcohol to be exchanged (such an alkoxide may be made in situ by adding an active metal such as Li, Na, K to the alcohol).

A typical procedure would be to react the α-campholenic alcohol or dihydro-α-campholenic alcohol with a large excess of the, e.g. methyl ester of the appropriate acid.

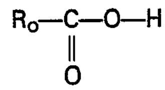

III'

wherein $R_o$ is as above, in the presence of catalytic amounts of, e.g. sodium methylate.

The compounds of formula I may be mixed with other flavouring materials to prepare flavour compositions which can be used to improve, enhance or modify the flavour of foodstuffs, or luxury consumables (e.g. tobacco products). The compounds could be added directly to said foodstuffs and luxury consumables, but it is normally the practice of the art to prepare a flavour mixture which is then added to the final product. Such foodstuffs and luxury consumables include, but are not limited to, gums, candies, jellies, gelatins, soft drinks (carbonated and non-carbonated), desserts, liquors, yogurts, teas, tobaccos, tobacco products and the like.

The compounds of formula I have properties which make them useful for improving, enhancing or modifying the organoleptic impression of flavour compositions, foodstuffs and luxury consumables. Although each of the compounds of formula I has its own unique and characteristic flavour, all of these compounds can be described as fruity and are preferably used to improve, enhance or modify the fruity character of a flavour. The α-campholenic alcohol is of foremost preference because it is distinctly superior to the other compounds and has an outstanding ability to provide "naturalness" to flavours. Next in preference is the dihydro-α-campholenic alcohol which is somewhat similar to the α-campholenic alcohol although slightly weaker in strength.

The esters of formula I all have flavours which are of a general fruity character with distinguishable berry notes and, in some instances, with floral topnotes. They are generally useful for imparting fruity notes and for enhancing the "naturalness" of a fruity flavour. The compound 2-(2,2,3-trimethylcyclopentan-1-yl)ethyl 2-methylpropionate possesses a natural fruit flavour and strong berry-like notes and is the preferred ester of formula I.

The alcohols of formula I, the especially preferred compounds of this invention, are characterized by particularly well defined sweet, natural, specific berry-like notes. Camphoraceous middle notes and woody backnotes also make important contributions to the flavour character of these compounds. These alcohols have a unique ability to improve or enhance the natural quality of fruity notes in general and are particularly valuable for the natural berry notes they can contribute. Because of these unique berry notes, the alcohols find special application in formulating berry flavours, especially strawberry, raspberry and black raspberry. These alcohols have also been found to be particularly useful in citrus flavours wherein they contribute a juicy top note to the flavour, i.e. make it taste more natural and more reminiscent of the natural juice flavour. As noted previously, of the two alcohols, α-campholenic alcohol is the somewhat stronger flavourant and is especially preferred for that reason.

A number of examples are provided herein to illustrate the utility of the compounds of this invention in fruit flavours. For example, when the α-campholenic alcohol was used in a raspberry flavour, the flavour was found to be rounder, more uniformly blended, more berry-like and more reminiscent of the taste of the natural berries. Similar effects were found when the compound was used in a strawberry flavour. When α-campholenic alcohol was incorporated into an orange flavour, the flavour with the compound present was considered to be better blended, rounder and more reminiscent of the natural fruit than the flavour without the compound. The flavour containing the compound was considered "more juicy", i.e. gave an impression more reminiscent of the natural orange juice.

The compounds of formula I are also particularly useful for improving the organoleptic properties of tobacco and tobacco products, e.g. tobaccos themselves, tobacco by-products such as reconstituted and homogenized leaf and stem, tobacco surrogates such as lettuce and cabbage leaf, materials used in tobacco processing such as paper, filters etc. and flavouring substance compositions used for tobacco products.

The use of α-campholenic alcohol is illustrated in the examples by its incorporation into a tobacco blend consisting of bright and burly tobaccos, stems and reconstituted leaf. When the treated cigarettes were compared with untreated cigarettes the former was found to smoke smoother and provide a more pleasant taste. The addition of the compound enhanced the "fullness" of the smoke and the feeling of moistness in the mouth.

When preparing flavour compositions, the compounds of formula I are preferably used in the range of about 0.5% to about 20% by weight of the flavour imparting ingredients with about 2% to about 10% being

especially preferred. In the finished foodstuff to be flavoured, the compounds of formula I are preferably present at a level of about 0.1 ppm to about 200 ppm with about 1 ppm to about 30 ppm being especially preferred. In the flavouring of tobacco products, the preferred range would be between about 100 ppm to about 250 ppm of the tobacco or tobacco surrogate used with about 175 ppm to about 225 ppm being especialy preferred. While the above are the preferred amounts contemplated, it should be understood that the amount and manner of use are dependent on the skill and preference of the flavourist and that greater amounts could be used to create special effects.

The claims are to be understood as not encompassing the use of natural materials which may contain a compound of this invention along with many other compounds of said natural materials and which have not been processed for the purpose of increasing the concentration of a compound of this invention to a point where the processed material can be used as a substitute for said compound contained therein.

The expression "substantially pure" as used below in the claims is thus used herein to mean, in particular, a compound of formula I which is free from accompanying substances which are present besides compounds of formula I in natural extracts. As substantially pure compounds of formula I in the scope of the present invention there should be understood, in particular, synthetically manufactured compounds of formula I.

The following examples are provided to illustrate the preferred embodiments as they are disclosed herein and are not to be construed as limiting.

Infrared spectra (ir) were recorded as neat samples on a Perkin-Elmer Model 457 spectrophotometer and absorptions are reported in inverse centimeters ($cm^{-1}$).

Molecular weights were determined on a Finnigan Model 4000 mass spectrometer.

Nuclear magnetic resonance (nmr) spectra were recorded as solutions in chloroform-$d_1$, using a Varian EM—360 proton spectrometer ($^1H$-nmr) and a Varian Model CFT—20 heteronuclear spectrometer ($^{13}C$-nmr), and are reported as δ units relative to tetramethylsilane (TMS) (0.0δ).

Gas-liquid chromatography (glc) was carried out on a 10% Carbowax®—20M (6 ft × $\frac{1}{4}$ in. (1 in. = 2,54 cm, 1 ft. = 30,48 cm)) column using a Varian Model 2700 gas chromatograph with thermal conductivity detector (TC).

Unless otherwise indicated weights are in grams, temperatures are in degrees centrigrade, pressures are in mm Hg and yields are based on theory.

## Example I

This example illustrates the preparation of compounds of formula I.

A. *Preparation of α-campholenic alcohol[2-(2,2,3-trimethylcyclopent-3-en-1-yl)ethanol]*

1. To a stirred suspension of 76 g (2.0 moles) of sodium borohydride, 800 ml of ethanol, 200 ml of water and 15 ml of 30% aqueous sodium hydroxide solution was added 608 g (4.0 moles) of α-campholenic aldehyde (prepared according to U.S. Patent 4,052,341 Example I) over 1.0 hour at 25—35°C. The resultant mixture was stirred at room temperature for an additional 5.0 hours, and subsequently quenched by pouring it into a stirred mixture of 600 ml of water and 25 ml of 30% aqueous sodium hydroxide solution. The mixture was allowed to settle and the lower aqueous layer was separated off and was extracted with two 200-ml portions of hexane. The oil layer and hexane extracts were combined and were washed with two 400-ml portions of water. The hexane layer was dried over anhydrous magnesium sulfate, filtered and the solvent was removed by atmospheric distillation.

The remaining oil was distilled at reduced pressure (0.6 mm Hg) to yield 544.1 g (88.3% yield) of α-campholenic alcohol: bp 72—76°C/0.5 mm Hg; $n_D^{20}$ 1.4728; mol wt. 154 (ms); nmr ($^1H$) 0.8 δ (3H, s), 1.0 (3H, s), 1.6 (3H, broad s, olefinic $CH_3$), 1.5—2.5 (5H, broad complex), 3.1 (1H, broad s, $D_2O$ exchangable, hydroxyl H), 3.7 (2H, t, J~7Hz), 5.3 (1H, broad multiplet, olefinic H); ir 3330 $cm^{-1}$ (—OH), 3035, 800, no carbonyl absorption in the 1650—1750 $cm^{-1}$ region; flavour: sweet, berry, camphoraceous, with woody backnote.

2. Using the procedure of U.S. Patent 4,052,341 Example I, (−)-α-pinene [$(\alpha)_D^{20}$ — 40°0′] was converted to (S)-α-campholenic aldehyde which was reduced using sodium borohydride to (S)-α-campholenic alcohol [$(\alpha)_{300}^{20}$ — 47.41°].

In an identical fashion, (+)-α-pinene [$(\alpha)_D^{20}$ + 40°0′] was converted to (R)-α-campholenic aldehyde which was reduced to (R)-α-campholenic alcohol [$(\alpha)_{300}^{20}$ + 47.42°].

A comparison of the ORD curves of the (R)- and (S)-aldehydes showed them to be mirror images as was the case with the (R)- and (S)-alcohols.

The flavour description of each isomer was similarly sweet, berry, camphoraceous.

B. *Preparation of esters of α-campholenic alcohol — general procedure*

A mixture of 46.2 g (0.30 mole) of α-campholenic alcohol, 75 ml of toluene, 0.1 g of p-toluenesulfonic acid monohydrate and 0.33 mole (10% excess) of the appropriate carboxylic acid was refluxed for 6.0 hours in a reaction flask (250-ml) equipped with a Dean-Stark water separator, during which time the theoretical amount of water was condensed from the distillate. The mixture was cooled and was poured into 100 ml of stirred 10% aqueous sodium carbonate solution. The upper toluene layer was separated and was washed with three 100-ml portions of water. The toluene was removed by distillation and the residual oil was purified by fractional distillation to give the following esters.

*Acetate:* 90.6% yield; bp 65—70°C/1.6 mm Hg; mol wt 196 (ms); ir 1745 $cm^{-1}$; flavour: fruity, berry, woody, floral backnote.

*Propionate:* 93.0% yield; bp 75—77°C/0.7 mm Hg; mol wt 210 (ms); ir 1740 $cm^{-1}$; flavour: fruity, berry, slightly camphoraceous, green.

*n-Butyrate:* 89.7% yield; bp 80—84°C/0.6 mm Hg; mol wt 224 (ms); ir 1740 $cm^{-1}$; flavour: floral, slightly fruity, camphoraceous, berry, slightly woody.

*2-Methylpropionate:* 88.4% yield; bp 77—79°C/0.5 mm Hg; mol wt 224 (ms); ir 1735 $cm^{-1}$; flavour: fruity, slightly green, berry, wood backnote.

The $^1H$ and $^{13}C$-nmr data were consistent with the individual structures of each ester.

C. *Preparation of dihydro-α-campholenic alcohol [2-(2,2,3-trimethylcyclopentan-1-yl)ethanol]*

A mixture of 77 g (0.5 mole) of α-campholenic alcohol, 1.54 g (2 wt-%) of dry palladium on carbon and 75 ml of ethanol was hydrogenated at 26—48°C and 40—64 psi hydrogen pressure (1 psi = 0,068 atm.) until hydrogen uptake ceased. The mixture was filtered to remove the catalyst and the solvent was removed from the filtrate by atmospheric distillation. The residual oil was fractionally distilled to yield dihydro-α-campholenic alcohol: 74.6 g (95.6% yield); bp 69—74°C/0.7 mm Hg; mol wt 156 (ms); nmr ($^1H$) 0.5δ (3H, s), 0.9 (6H, overlapping s and d), 1.0—2.0 (8H, broad complex), 3.1 (1H, broad s, $D_2O$ exchangable, hydroxyl H), 3.6 (2H, broad multiplet, $-CH_2OH$); ir 3330 $cm^{-1}$ (—OH), no olefinic absorptions at 3025 or 800 $cm^{-1}$; flavour: berry, slightly camphoraceous, sweet, slightly fruity.

D. *Preparation of esters of dihydro-α-campholenic alcohol — general procedure*

A mixture off 39.0 g (0.25 mole) of dihydro-α-campholenic alcohol, 75 ml of toluene, 0.1 g of p-toluenesulfonic acid monohydrate and 0.30 mole (20% excess) of the appropriate carboxylic acid was refluxed for 5—6 hours in a reaction flask (250-ml) equipped with a Dean-Stark water separator, during which time the theoretical amount of water was condensed from the distillate. The mixture was cooled and was poured into 100 ml of stirred 10% aqueous sodium carbonate solution. The upper toluene layer was separated and was washed with three 100-ml portions of water. The toluene was removed by distillation and the residual oil was purified by fractional distillation to give the following esters:

*Acetate:* 92.3% yield; bp 72—78°C/0.9 mm Hg; mol wt 198 (ms); ir 1745 $cm^{-1}$; flavour: fruity, camphoraceous, prune-like.

*2-Methylpropionate:* 90.8% yield; bp 82—83°C/0.4 mm Hg; mol wt 226 (ms); ir 1740 $cm^{-1}$; flavour: fruity, berry, with woody backnotes.

The $^1H$ and $^{13}C$-nmr data were consistent with the individual structures of each ester.

## Example II

This example illustrates the use of the compounds of the invention as flavourants.

A. *Artificial berry flavours*

1. Raspberry flavour

| Constituents | Parts by weight |
|---|---|
| α-Ionone | 2.85 |
| β-Ionone | 2.25 |
| Ethyl acetate | 22.60 |
| Acetyl methyl carbinol (acetoin) | 28.34 |
| δ-Decalactone | 2.25 |
| Linalool | 4.50 |
| α-Terpineol | 2.25 |
| cis-3-Hexen-1-ol | 2.25 |
| n-Amyl alcohol | 4.50 |
| Caproic acid | 18.07 |
| Caprylic acid | 4.50 |
| | 94.36 |
| α-Campholenic alcohol | 5.64 |
| Total | 100.00 |

2. Strawberry flavour

| Constituents | Parts by weight |
|---|---|
| Acetic acid | 14.37 |
| Ethyl methylphenylglycidate | 0.85 |
| Caprylic acid | 2.85 |
| Diacetyl (butanedione) | 0.57 |
| Ethyl acetate | 26.00 |
| Ethyl butyrate | 11.40 |
| γ-Undecalactone | 1.40 |
| cis-3-hexen-1-ol | 1.14 |
| Maltol | 7.14 |
| Levulinic acid | 20.00 |
| Methyl 2-methylbutyrate | 7.14 |
| | 92.86 |
| α-Campholenic alcohol | 7.14 |
| Total | 100.00 |

Both of the above artificial berry flavours were prepared with and without α-campholenic alcohol as indicated. In each case, the presence of the α-campholenic alcohol made a significant difference. The flavour of the composition was rounded out, more berry-like and more reminiscent of a natural fruit flavour.

B. *Artificial orange flavour*

| Constituents | Parts by weight |
|---|---|
| Acetaldehyde | 1.0 |
| Ethyl butyrate | 1.0 |
| Decanal | 0.4 |
| Linalool | 0.5 |
| Orange oil Florida (cold pressed) | 95.1 |
| | 98.0 |
| α-Campholenic alcohol | 2.0 |
| Total | 100.00 |

The addition of α-campholenic alcohol to the above artificial orange flavour resulted in an improved composition. The presence of the alcohol increased the juiciness, rounded out the flavour and added to the natural notes.

Effects similar to those noted above for the addition of α-campholenic alcohol to flavour compositions can also be achieved with the use of dihydro-α-campholenic alcohol with perhaps an adjustment of the amount used.

C. *Tobacco product*

A standard cigarette blend was prepared as described below:

| Constituents | | Parts by weight |
|---|---|---|
| Bright tobacco | | 55 |
| Burley tobacco | | 25 |
| Expanded stems | | 5 |
| Reconstituted leaf | | 15 |
| | Total | 100 |

α-Campholenic alcohol (200 ppm by weight) was added to a portion of the blend and cigarettes were prepared with and without the additive and compared by smoking. The cigarettes containing the additive were markedly improved over those without it. The smoke was considerably smoother and more pleasant. The moistness of the mouth was markedly increased and the mouth feel or "fullness" of the smoke was enhanced.

**Claims**

1. A flavour composition comprising a compound of the formula

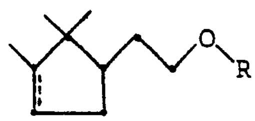

I

wherein the dotted line represents an optional bond, and R represents hydrogen or an alkanoyl group of two to five carbon atoms.

2. A flavour composition according to claim 1, comprising a compound of the formula

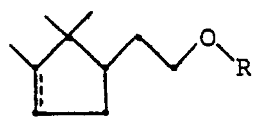

I

wherein the dotted line represents an optional bond, and R represents hydrogen or an alkanoyl group of two to five carbon atoms,
in substantially pure form or in form of mixtures, with the exception of natural mixtures containing compounds of formula I.

3. A composition according to claim 1 or 2 wherein R is hydrogen.

4. A composition according to claim 3 wherein the compound is 2-(2,2,3-trimethylcyclopent-3-en-1-yl)-ethanol.

5. A composition according to claim 3 wherein the compound is 2-(2,2,3-trimethylcyclopentan-1-yl)-ethanol.

6. A composition according to claim 1 or 2 wherein the compound is 2-(2,2,3-trimethylcyclopentan-1-yl)ethyl 2-methylpropionate.

7. A foodstuff comprising a compound of the formula

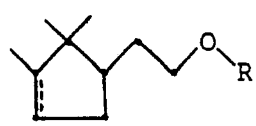

I

wherein the dotted line represents an optional bond, and R represents hydrogen or an alkanoyl group of two to five carbon atoms.

8. A foodstuff according to claim 7, comprising a compound of the formula

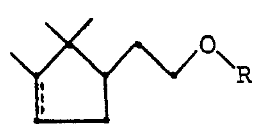

I

wherein the dotted line represents an optional bond, and R represents hydrogen or an alkanoyl group of two to five carbon atoms,
in substantially pure form or in form of mixtures, with the exception of natural mixtures containing compounds of formula I.

9. A foodstuff according to claim 7 or 8 wherein R is hydrogen.

10. A foodstuff according to claim 9 wherein the compound is 2-(2,2,3-trimethylcyclopent-3-en-1-yl)-ethanol.

11. A foodstuff according to claim 9 wherein the compound is 2-(2,2,3-trimethylcyclopentan-1-yl)-ethanol.

12. A foodstuff according to claim 7 or 8 wherein the compound is 2-(2,2,3-trimethylcyclopentan-1-yl)-ethyl 2-methylpropionate.

13. A tobacco product comprising a compound of the formula

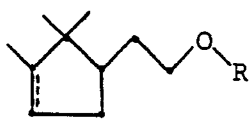

I

wherein the dotted line represents an optional bond, and R represents hydrogen or an alkanoyl group of two to five carbon atoms.

14. A tobacco product according to claim 13, comprising a compound of the formula

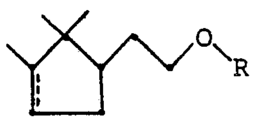

I

wherein the dotted line represents an optional bond, and R represents hydrogen or an alkanoyl group of two to five carbon atoms,
in substantially pure form or in form of mixtures, with the exception of natural mixtures containing compounds of formula I.

15. A tobacco product according to claim 13 and 14 wherein R is hydrogen.

16. A tobacco product according to claim 15 wherein the compound is 2-(2,2,3-trimethylcyclopent-3-en-1-yl)ethanol.

17. A tobacco product according to claim 15 wherein the compound is 2-(2,2,3-trimethylcyclopentan-1-yl)ethanol.

18. A tobacco product according to claim 13 or 14 wherein the compound is 2-(2,2,3-trimethylcyclopentan-1-yl)ethyl 2-methylpropionate.

19. A method for improving the flavour of a flavour composition which comprises adding thereto a compound of the formula

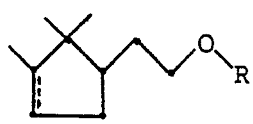

I

wherein the dotted line represents an optional bond, and R represents hydrogen or an alkanoyl group of two to five carbon atoms.

20. A method for improving the flavour or a flavour composition according to claim 19, which comprises adding thereto a compound of the formula

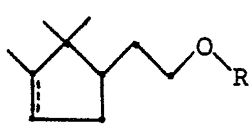

I

wherein the dotted line represents an optional bond, and R represents hydrogen or an alkanoyl group of two to five carbon atoms,
in substantially pure form or in form of mixtures, with the exception of natural mixtures containing compounds of formula I.

21. The method of claim 19 or 20 wherein R is hydrogen.

22. The method of claim 21 wherein the compound is 2-(2,2,3-trimethylcyclopent-3-en-1-yl)ethanol.

23. The method of claim 21 wherein the compound is 2-(2,2,3-trimethylcyclopentan-1-yl)ethanol.

24. The method of claim 19 or 20 wherein the compound is 2-(2,2,3-trimethylcyclopentan-1-yl)ethyl 2-methylpropionate.

25. The use of a compound of the formula

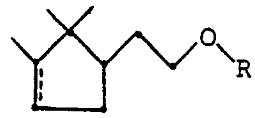

I

wherein the dotted line represents an optional bond, and R represents hydrogen or an alkanoyl group of two to five carbon atoms,
as flavourants.

26. The use according to claim 25 of a compound of the formula

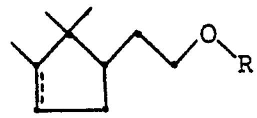

I

wherein the dotted line represents an optional bond, and R represents hydrogen or an alkanoyl group of two to five carbon atoms,
in substantially pure form or in form of mixtures, with the exception of natural mixtures containing compounds of formula I.

27. A compound of the formula

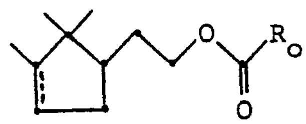

I'

wherein the dotted line represents an optional bond, and $R_o$ is an alkyl group of two to four carbon atoms.

28. 2-(2,2,3-Trimethylcyclopentan-1-yl)ethyl 2-methylpropionate.

29. Process for the manufacture of the compounds of formula

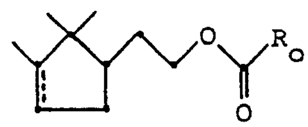

I'

wherein the dotted line represents an optional bond, and $R_o$ is an alkyl group of two to four carbon atoms, comprising esterifying an alcohol of the formula

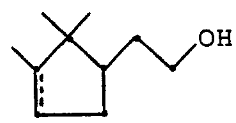

II

wherein the dotted line is as given above,
or trans-esterifying an ester

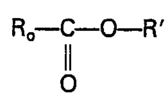

III

wherein $R_o$ is as above and R′ is an alkyl from one to five carbons,
by reacting with an alcohol of structure II.

**Patentansprüche**

1. Geschmackstoffkomposition, enthaltend eine Verbindung der Formel

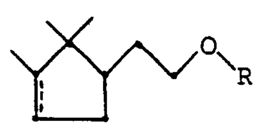

I

worin die gestrichelte Linie eine fakultative Bindung darstellt und R Wasserstoff oder einen Alkanoylrest mit 2 bis 5 Kohlenstoffatomen bedeutet.

2. Geschmackstoffkomposition gemäss Anspruch 1, enthaltend eine Verbindung der Formel

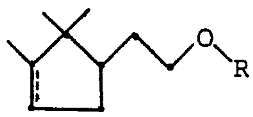

I

worin die gestrichelte Linie eine fakultative Bindung darstellt und R Wasserstoff oder einen Alkanoylrest mit 2 bis 5 Kohlenstoffatomen bedeutet,
in im wesentlichen reiner Form oder in Form von Gemischen, mit der Ausnahme von natürlichen, Verbindungen der Formel I enthaltenden Gemischen.

3. Komposition gemäss Anspruch 1 oder 2, worin R Wasserstoff ist.

4. Komposition gemäss Anspruch 3, worin die Verbindung 2-(2,2,3-Trimethylcyclopent-3-en-1-yl)-aethanol ist.

5. Komposition gemäss Anspruch 3, worin die Verbindung 2-(2,2,3-Trimethylcyclopentan-1-yl)aethanol ist.

6. Komposition gemäss Anspruch 1 oder 2, worin die Verbindung 2-(2,2,3-Trimethylcyclopentan-1-yl)-aethyl-2-methylpropionat ist.

7. Nahrungsmittel, enthaltend eine Verbindung der Formel

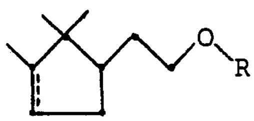

I

worin die gestrichelte Linie eine fakultative Bindung darstellt und R Wasserstoff oder einen Alkanoylrest mit 2 bis 5 Kohlenstoffatomen bedeutet.

8. Nahrungsmittel gemäss Anspruch 7, enthaltend eine Verbindung der Formel

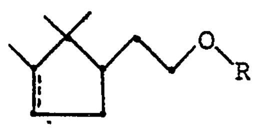

I

worin die gestrichelte Linie eine fakultative Bindung darstellt und R Wasserstoff oder einen Alkanoylrest mit 2 bis 5 Kohlenstoffatomen bedeutet,
in im wesentlichen reiner Form oder in Form von Gemischen, mit der Ausnahme von natürlichen, Verbindungen der Formel I enthaltenden Gemischen.

9. Nahrungsmittel gemäss Anspruch 7 oder 8, worin R Wasserstoff bedeutet.

10. Nahrungsmittel gemäss Anspruch 9, worin die Verbindung 2-(2,2,3-Trimethylcyclopent-3-en-1-yl)-aethanol ist.

11. Nahrungsmittel gemäss Anspruch 9, worin die Verbindung 2-(2,2,3-Trimethylcyclopentan-1-yl)-aethanol ist.

12. Nahrungsmittel gemäss Anspruch 7 oder 8, worin die Verbindung 2-(2,2,3-Trimethylcyclopentan-1-yl)aethyl-2-methylpropionat ist.

13. Tabakprodukt, enthaltend eine Verbindung der Formel

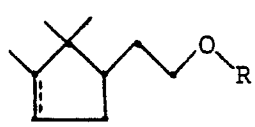

I

worin die gestrichelte Linie eine fakultative Bindung darstellt und R Wasserstoff oder einen Alkanoylrest mit 2 bis 5 Kohlenstoffatomen bedeutet.

14. Tabakprodukt gemäss Anspruch 13, enthaltend eine Verbindung der Formel

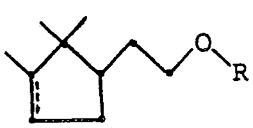

I

worin die gestrichelte Linie eine fakultative Bindung darstellt und R Wasserstoff oder einen Alkanoylrest mit 2 bis 5 Kohlenstoffatomen bedeutet,
in im wesentlichen reiner Form oder in Form von Gemischen, mit der Ausnahme von natürlichen, Verbindungen der Formel I enthaltenden Gemischen.

15. Tabakprodukt gemäss Anspruch 13 oder 14, worin R Wasserstoff ist.

16. Tabakprodukt gemäss Anspruch 15, worin die Verbindung 2-(2,2,3-Trimethylcyclopent-3-en-1-yl)-aethanol ist.

17. Tabakprodukt gemäss Anspruch 15, worin die Verbindung 2-(2,2,3-Trimethylcyclopentan-1-yl)-aethanol ist.

18. Tabakprodukt gemäss Anspruch 13 oder 14, worin die Verbindung 2-(2,2,3-Trimethylcyclopentan-1-yl)aethyl-2-methylpropionat ist.

19. Verfahren zum Verbessern des Geschmackes einer Geschmackstoffkomposition, dadurch gekennzeichnet, dass man dazu eine Verbindung der Formel

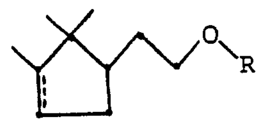

I

worin die gestrichelte Linie eine fakultative Bindung und R Wasserstoff oder einen Alkanoylrest mit 2 bis 5 Kohlenstoffatomen bedeutet,
zufügt.

20. Verfahren zum Verbessern des Geschmackes einer Geschmackstoffkomposition gemäss Anspruch 19, dadurch gekennzeichnet, dass man eine Verbindung der Formel

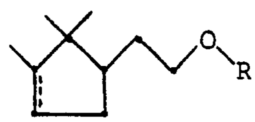

I

worin die gestrichelte Linie eine fakultative Bindung darstellt und R Wasserstoff oder einen Alkanoylrest mit 2 bis 5 Kohlenstoffatomen bedeutet,
in im wesentlichen reiner Form oder in Form von Gemischen, mit der Ausnahme von natürlichen, Verbindungen der Formel I enthaltenden Gemischen, zufügt.

21. Verfahren gemäss Anspruch 19 oder 20, worin R Wasserstoff bedeutet.

22. Verfahren gemäss Anspruch 21, worin die Verbindung 2-(2,2,3-Trimethylcyclopent-3-en-1-yl)-aethanol ist.

23. Verfahren gemäss Anspruch 21, worin die Verbindung 2-(2,2,3-Trimethylcyclopentan-1-yl)aethanol ist.

24. Verfahren gemäss Anspruch 19 oder 20, worin die Verbindung 2-(2,2,3-Trimethylcyclopentan-1-yl)-aethyl-2-methylpropionat ist.

25. Verwendung einer Verbindung der Formel

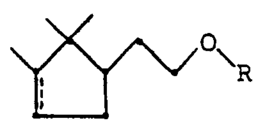

I

worin die gestrichelte Linie eine fakultative Bindung darstellt und R Wasserstoff oder einen Alkanoylrest mit 2 bis 5 Kohlenstoffatomen bedeutet,
in im wesentlichen reiner Form oder in Form von Gemischen, mit der Ausnahme von natürlichen, Verbindungen der Formel I enthaltenden Gemischen, als Geschmacksstoff.

26. Verwendung einer Verbindung der Formel

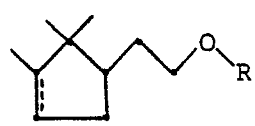

I

worin die gestrichelte Linie eine fakultative Bindung darstellt und R Wasserstoff oder einen Alkanoylrest mit 2 bis 5 Kohlenstoffatomen bedeutet,
in im wesentlichen reiner Form oder in Form von Gemischen, mit der Ausnahme von natürlichen, Verbindungen der Formel I enthaltenden Gemischen, gemäss Anspruch 25.

27. Eine Verbindung der Formel

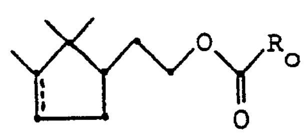

I'

worin die gestrichelte Linie eine fakultative Bindung darstellt und $R_o$ einen Alkylrest mit 2 bis 4 Kohlenstoffatomen bedeutet.

28. 2-(2,2,3-Trimethylcyclopentan-1-yl)aethyl-2-methylpropionat.

29. Verfahren zur Herstellung der Verbindungen der Formel

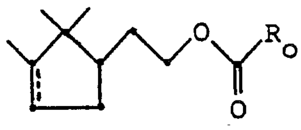

I'

worin die gestrichelte Linie eine fakultative Bindung darstellt und $R_o$ einen Alkylrest mit 2 bis 4 Kohlenstoffatomen bedeutet, dadurch gekennzeichnet, dass man einen Alkohol der Formel

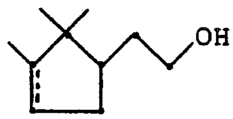

II

worin die gestrichelte Linie obige Bedeutung hat, verestert, oder dass man einen Ester

$$R_o-\underset{\underset{O}{\|}}{C}-O-R' \qquad III$$

worin $R_o$ die obige Bedeutung hat und R' Alkyl mit 1 bis 5 Kohlenstoffatomen bedeutet, durch Reaktion mit einem Alkohol der Formel II umestert.

**Revendications**

1. Une composition aromatisante comprenant un composé de formule

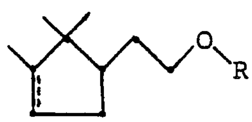

I

dans laquelle le trait interrompu représente une liaison facultative et R représente l'hydrogène ou un groupe alcanoyle en C 2—C 5.

2. Une composition aromatisante selon la revendication 1, comprenant un composé de formule

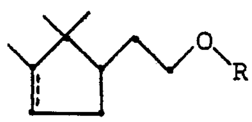

I

dans laquelle le trait interrompu représente une liaison facultative et R représente l'hydrogène ou un groupe alcanoyle en C 2—C 5, à l'état pur ou à l'état de mélanges, à l'exception des mélanges naturels contenant des composés de formule I.

3. Une composition selon la revendication 1 ou 2, dans laquelle R représente l'hydrogène.

4. Une composition selon la revendication 3, dans laquelle le composé est le 2-(2,2,3-triméthylcyclopenta-3-ène-1-yl)-éthanol.

5. Une composition selon la revendication 3, dans laquelle le composé est 2-(2,2,3-triméthylcyclopentane-1-yl)-éthanol.

6. Une composition selon la revendication 1 ou 2, dans laquelle le composé est le 2-méthylpropionate de 2-(2,2,3-triméthylcyclopentane-1-yl)-éthyle.

7. Un produit alimentaire comprenant un composé de formule

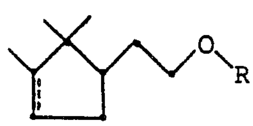

I

dans laquelle le trait interrompu représente une liaison facultative et R représente l'hydrogène ou un groupe alcanoyle en C 2—C 5.

8. Un produit alimentaire selon la revendication 7, comprenant un composé de formule

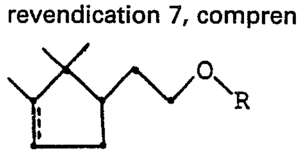

I

dans laquelle le trait interrompu représente une liaison facultative et R représente l'hydrogène ou un groupe alcanoyle en C 2—C 5,
à l'état pur ou à l'état de mélanges, à l'exception des mélanges naturels contenant des composés de formule I.

9. Un produit alimentaire selon la revendication 7 ou 8, dans laquelle R représente l'hydrogène.

10. Un produit alimentaire selon la revendication 9, dans lequel le composé est le 2-(2,2,3-triméthylcyclopenta-3-ène-1-yl)-éthanol.

11. Un produit alimentaire selon la revendication 9, dans lequel le composé est 2-(2,2,3-triméthylcyclopentane-1-yl)-éthanol.

12. Un produit alimentaire selon la revendication 7 ou 8, dans lequel le composé est le 2-méthylpropionate de 2-(2,2,3-triméthylcyclopentane-1-yl)-éthyle.

13. Un produit de tabac comprenant un composé de formule

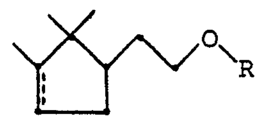 I

dans laquelle le trait interrompu représente une liaison facultative et R représente l'hydrogène ou un groupe alcanoyle en C 2—C 5.

14. Un produit de tabac selon la revendication 13, comprenant un composé de formule

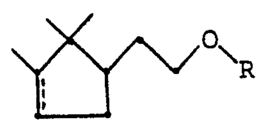 I

dans laquelle le trait interrompu représente une liaison facultative et R représente l'hydrogène ou un groupe alcanoyle en C 2—C 5,
à l'état pur ou à l'état de mélanges, à l'exception des mélanges naturels contenant des composés de formule I.

15. Un produit de tabac selon la revendication 13 ou 14, dans lequel R représente l'hydrogène.

16. Un produit de tabac selon la revendication 15, dans lequel le composé est le 2-(2,2,3-triméthylcyclopenta-3-ène-1-yl)-éthanol.

17. Un produit de tabac selon la revendication 3, dans lequel le composé est le 2-(2,2,3-triméthylcyclopentane-1-yl)-éthanol.

18. Un produit de tabac selon la revendication 13 ou 14, dans lequel le composé est le 2-méthylpropionate de 2-(2,2,3-triméthylcyclopentane-1-yl)-éthyle.

19. Un procédé pour améliorer l'arôme d'une composition aromatisante qui consiste à lui ajouter un composé de formule

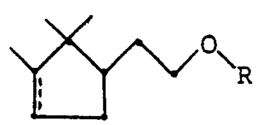 I

dans laquelle le trait interrompu représente une liaison facultative et R représente l'hydrogène ou un groupe alcanoyle en C 2—C 5.

20. Un procédé pour améliorer l'arôme d'une composition aromatisante selon la revendication 19, qui consiste à lui ajouter un composé de formule

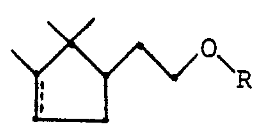 I

dans laquelle le trait interrompu représente une liaison facultative et R représente l'hydrogène ou un groupe alcanoyle en C 2—C 5,
à l'état pur ou à l'état de mélanges, à l'exception des mélanges naturels contenant des composés de formule I.

21. Le procédé de la revendication 19 ou 20, dans lequel R représente l'hydrogène.

22. Le procédé de la revendication 21, dans lequel le composé est le 2-(2,2,3-triméthylcyclopenta-3-ène-1-yl)-éthanol.

23. Le procédé de la revendication 21, dans lequel le composé est le 2-(2,2,3-triméthylcyclopentane-1-yl)-éthanol.

24. Le procédé de la revendication 19 ou 20, dans lequel le composé est le 2-méthylpropionate de 2-(2,2,3-triméthylcyclopentane-1-yl)-éthyle.

25. L'utilisation d'un composé de formule

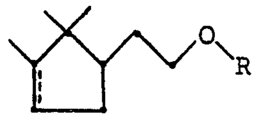

I

dans laquelle le trait interrompu représente une liaison facultative et R représente l'hydrogène ou un groupe alcanoyle en C 2—C 5,
en tant que substance aromatisante.

26. L'utilisation selon la revendication 25 d'un composé de formule

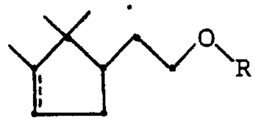

I

dans laquelle le trait interrompu représente une liaison facultative et R représente l'hydrogène ou un groupe alcanoyle en C 2—C 5,
à l'état pur ou à l'état de mélanges, à l'exception des mélanges naturels contenant des composés de formule I.

27. Un composé de formule

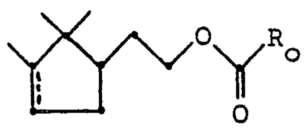

I'

dans laquelle le trait interrompu représente une liaison facultative et $R_o$ représente un groupe alkyle en C 2—C 4.

28. Le 2-méthylpropionate de 2-(2,2,3-triméthylcyclopentane-1-yl)-éthyle.

29. Procédé de préparation des composés de formule

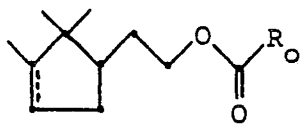

I'

dans laquelle le trait interrompu représente une liaison facultative et $R_o$ représente un groupe alkyle en C 2—C 4, qui consiste à estérifier un alcool de formule

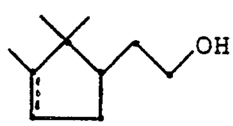

II

dans laquelle le trait interrompu a la signification indiqué ci-dessus,
ou à transestérifier un ester de formule

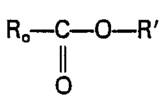

III

dans laquelle $R_o$ a les significations indiquées ci-dessus et R' représente un groupe alkyle en C 1—C 5, par réaction avec un alcool de formule II.